# EUROPÄISCHE PATENTANMELDUNG

(11) **EP 0 650 742 A1**
(43) Veröffentlichungstag der Anmeldung: **03.05.1995**
(21) Anmeldenummer: 94117103.5
(22) Anmeldetag: 28.10.1994
(51) Int. Cl.: A61N 1/39

(54) **Verfahren und Vorrichtung zum Betreiben eines Defibrillators**

(30) Priorität: 29.10.1993 DE 4337110
(71) Anmelder: Dietl, Michael A.J., D-82266 Inning (DE)
(72) Erfinder: Dietl, Michael A.J., D-82266 Inning (DE)
(74) Vertreter: Bohnenberger, Johannes, Dr.

(57) **Zusammenfassung**

Die Erfindung betrifft ein Verfahren und eine Vorrichtung zum effizienten Betreiben eines Defibrillators (1), wobei der als halbautomatische Einrichtung ausgebildete Defibrillator (1) über ein im Vorwärtspfad betriebenes neuronales Netzwerk verfügt und die Netzwerkparameter auf einer auswechselbaren Speicherkarte (3) abgelegt sind und die Speicherkarte zur temporären Kommunikation mit einem externen Personalcomputer (4) benutzt wird. Die Speicherkarte (3) enthält des weiteren während des Einsatzes des Defibrillators aufgenommene Elektrokardiogramme, welche gemeinsam mit den Netzwerkparametern zum Personalcomputer (4) übertragen werden. Automatisch oder manuell bewertete Elektrokardiogramme werden in eine Entwicklungsdatenbank (8) des Personalcomputers (4) übernommen, wobei diese übernommenen Elektrokardiogramme zur Durchführung eines Lernprozesses, d.h. des Betreibens eines neuronalen Netzes im Rückwärtspfad, im Personalcomputer (4) verwendet werden. Im Ergebnis des Lernprozesses entstehen neue Netzwerkparameter, die mit Hilfe eines Defibrillator-Simulationsprogramms und unter Nutzung von Daten einer personalcomputerinternen Referenz- bzw. Standarddatenbank auf Effizienz geprüft werden. Im Falle einer positiven Effizienzprüfung werden die neuen, aktualisierten und so optimierten Netzwerkparameter auf die Speicherkarte (3) zurückgeschrieben und zum Defibrillator (1) übertragen, derart, daß beim nachfolgenden Einsatz das im Defibrillator (1) vorhandene neuronale Netzwerk mit optimierten Netzwerkparametern arbeitet.

## Beschreibung

Die Erfindung betrifft ein Verfahren zum Betreiben eines Defibrillators und eine Vorrichtung zum Durchführen eines derartigen Verfahrens gemäß dem Oberbegriff der Patentansprüche 1 beziehungsweise 5.

Insbesondere zur Betreuung von Herzpatienten sind seit vielen Jahren Defibrillatoren bekannt. Mit Hilfe derartiger Defibrillatoren wird zunächst über entsprechende Elektroden ein Elektrokardiogramm des zu behandelnden Patienten aufgenommen. Mit Hilfe spezieller Bewertungsalgorithmen erfolgt eine Auswertung der über eine bestimmte Zeit einlaufenden Elektrokardiogramme dahingehend, ob ein Defibrillator-Impuls zum Aktivieren, Stimulieren oder Stabilisieren der Herztätigkeit des Patienten mit festgestellter Arrhythmie erforderlich ist. Eine Aktivierung oder Wiederaktivierung beziehungsweise Stabilisierung der Herztätigkeit erfolgt dann durch mittels des Defibrillators pulsgesteuerte Spannungsstöße, die über Elektroden, z.B. Klebeelektroden, welche anterior-anterior plaziert sind, auf den Körper beziehungsweise das Herz des Patienten einwirken.

Die Kriterien für das Auslösen eines Defibrillatorpulses beziehungsweise der Elektrodenspannungseinwirkung erfolgt durch Beurteilung des Herzkammerflimmerns hinsichtlich Amplitude und zeitbezogener Parameter. Um die Sicherheit bei der Bewertung von auftretendem Kammerflimmern und gegebenenfalls nachfolgendem Auslösen eines Defibrillatorpulses zu erhöhen, ist es bekannt, den Defibrillator mit einem speziellen Artefaktenentdeckungssystem auszurüsten, wobei die Auswertung verzerrter EKGs ausgeschlossen und ein höherer Genauigkeitsgrad bei der Bewertung erzielt wird.

Da die bekannten Defibrillatoren über die Möglichkeit nachträglicher Datenüberprüfungen verfügen müssen, sind Vorrichtungen zur Ereignisdokumentation, d.h. zum Aufzeichnen aufgenommener Elektrokardiogramme und gegebenenfalls von zusätzlichen Informationen, vorhanden, welche z.B. auf einem Magnetband abgespeichert werden. Nach der Behandlung beziehungsweise dem Einsatz des Defibrillators wird dann das Aufzeichnungsmaterial vom Arzt bewertet und eine nachträgliche Beurteilung des Notfallsystems durchgeführt.

Neben Magnetbandkassettenspeichersystemen sind wiederverwendbare Speichermodule in Form von Speicherkarten für halbautomatische EKG-Aufzeichnungen bekannt.

Bekannte Defibrillatoren sind beispielsweise der Defibrillator Kardio-AID-LS der Firma S&W Medientecnic A/S oder der LIFEPAK 300 der Firma Physio Control.

Aus der europäischen Patentanmeldung EP 0 465 241 A2 ist eine Vorrichtung und ein Verfahren zur Feststellung von Herzrhythmusstörungen unter Verwendung eines neuronalen Netzwerkes bekannt.

Bei der dort gezeigten implantierbaren Vorrichtung wird der Herzrhythmus des Herzens laufend überwacht, und mit Hilfe eines Mikroprozessors, der mit einem neuronalen Netzwerk zusammenwirkt, werden auftretende Herzrhythmusstörungen bewertet.

Durch das dort gezeigte, selbstlernende neuronale Netzwerk soll die Genauigkeit und Sicherheit bei der Erkennung von Herzrhythmusstörungen verbessert und die Bewertungszeit verkürzt werden.

Nachdem eine Herzrhythmusstörung diagnostiziert wurde, wird ein ebenfalls implantierter Defibrillator aktiv und löst über eine Defibrillatorelektrode einen Impuls mit dem Ziel der Stabilisierung der Herztätigkeit aus.

Problematisch bei der dort gezeigten Vorrichtung ist die Tatsache, daß der Lernprozeß des neuronalen Netzwerkes nicht von außen steuerbar ist, so daß unerwünschte oder gar falsche Diagnosen getroffen und das Auslösen eines Defibrillatorschocks zum falschen Zeitpunkt erfolgt. Bei der dort gezeigten Vorrichtung können also weder die Effizienz des selbstlernenden neuronalen Netzwerkes überprüft noch effizienzvermindernde Netzwerkparameter verworfen werden.

Es ist daher Aufgabe der Erfindung, ein Verfahren und eine Vorrichtung zum Betreiben des Defibrillators anzugeben, wobei zum einen die Vorteile eines neuronalen Netzes beziehungsweise Netzwerkes zur Analyse aufgenommener Elektrokardiogramme genutzt werden können und andererseits kontrolliert ein effizienter Lernprozeß durch Bereitstellung neuer, optimierter Netzwerkparameter für das neuronale Netz innerhalb eines Defibrillators möglich wird. Die Vorrichtung zum effizienteren Betreiben eines Defibrillators soll so aufgebaut sein, daß eine unsachgemäße Bedienung oder die Übernahme unautorisierter veränderter Netzwerkparameter nicht möglich ist, wodurch die hohen Anforderungen an die Einsatzsicherheit und Funktionstüchtigkeit des Defibrillators erfüllt werden.

Die Lösung der Aufgabe der Erfindung erfolgt mit den Merkmalen der Patentansprüche 1 und 5, wobei die Unteransprüche mindestens zweckmäßige Ausgestaltungen und Weiterbildungen des Grundgedankens der Erfindung zeigen.

Dieser Grundgedanke der Erfindung besteht darin, einen Lernprozeß eines neuronalen Netzwerkes kontrolliert und optimiert anhand sicherer Bewertungskriterien durchführen zu können, wobei die Möglichkeit gegeben ist, die Effizienz des neuronalen Netzwerkes beziehungsweise des Netzwerkes mit aktualisierten Netzwerkparametern durch Simulation zu prüfen, um zu verhindern, daß effizienzverringernde Netzwerkparameter in eine entsprechende, mikrocontrollergesteuerte Bewertungseinheit des Defibrillators gelangen.

Hierfür wirken ein oder mehrere Defibrillatoren, welche über eine Schnittstelle beziehungsweise eine Lese/Schreibeinrichtung verfügen, temporär z.B. über eine auswechselbare Speicherkarte mit einem Personalcomputer zusammen. Dieser Personalcomputer wird nunmehr für einen externen Lernprozeß des neuronalen Netzwerkes, welches im Defibrillator enthalten ist, und über kennzeichnende Netzwerkparameter verfügt, durchgeführt.

Die ohnehin für statistische Zwecke abzuspeichernden, aktuell aufgenommenen Elektrokardiogramme werden mit Hilfe der Lese/Schreibeinheit und der auswechselbaren Speicherkarte abgespeichert und zum Personalcomputer über eine entsprechende Schnittstelle beziehungsweise Lese/Schreibeinheit transferiert. Gleichzeitig enthält die auswechselbare Speicherkarte die jeweils im Defibrillator benutzten Netzwerkparameter des neuronalen Netzes. Auch diese Daten gelangen zum Personalcomputer. Der Personalcomputer verfügt über eine interne Entwicklungsdatenbank, welche, zweckmäßigerweise nach ärztlicher Bewertung der aufgenommenen Kardiogramme, die bewerteten Kardiogramme übernimmt.

Es liegt im Sinne der Erfindung, daß der Personalcomputer über einen komplexen Algorithmus zur automatischen Bewertung der über die Speicherkarte bereitgestellten Elektrokardiogramme verfügt, wobei diese Bewertung unter dem Gesichtspunkt durchgeführt wird, ob aus dem Verlauf des Kardiogramms das Auslösen eines Defibrillatorschocks sinnvoll und notwendig ist. Dadurch, daß eine Vielzahl von im Einsatz befindlichen Defibrillatoren temporär über einen Austausch der Speicherkarte mit nur einem einzigen Personalcomputer kommunizieren, muß die erforderliche Soft- und Hardware zur Realisierung des Bewertungsalgorithmus nur einmal, und damit kostengünstig, zur Verfügung stehen.

Mit den nunmehr in der Entwicklungsdatenbank des Personalcomputers befindlichen aktualisierten Daten werden die ebenfalls über die Speicherkarte anliegenden Netzwerkparameter durch einen Lernprozeß des im Rückwärtspfad betriebenen, im PC abgebildeten neuronalen Netzes optimiert.

Die somit optimierten Netzwerkparameter werden mit einem ebenfalls im Personalcomputer enthaltenen Defibrillatorsimulationsprogramm unter Nutzung von auf einer weiteren internen Datenbank, nämlich einer Referenz- beziehungsweise Standarddatenbank, gespeicherten Vergleichsdaten einer Effizienzprüfung unterzogen. Im Falle eines positiven Abschlusses der Effizienzprüfung werden die derart optimierten Netzwerkparameter auf die auswechselbare Speicherkarte zurückgeschrieben und damit über das Medium Speicherkarte hin zum Defibrillator beziehungsweise zu den Defibrillatoren (rück-)übertragen.

Durch die vorstehend geschilderte Verfahrenweise gelingt es, die ursprünglichen Netzwerkparameter des neuronalen Netzes im Defibrillator sukzessive einem Lernprozeß zu unterziehen, wobei ein besonders hoher Wirkungsgrad dann erzielt wird, wenn die Daten, d.h. Elektrokardiogramme mehrerer, im Einsatz befindlicher Defibrillatoren, auf die Entwicklungsdatenbank des Personalcomputers gelangen.

Durch die konsequente Trennung zwischen einem quasi neuronalen Anwendungsnetzwerk im Defibrillator und einem neuronalen lernfähigen Netzwerk im Personalcomputer wird eine unkontrollierte Veränderung der Arbeitsweise des neuronalen Netzes und damit eine möglicherweise eintretende Verschlechterung bei der Bewertung der Elektrokardiogramme mit Fehlauslösung eines Defibrillatorschocks verhindert, andererseits aber kann durch die Verlagerung des Lernprozesses in ein externes Gerät das zeitkritische Lernproblem beim Betreiben eines neuronalen Netzes im Rückwärtspfad minimiert werden.

Es liegt im Sinne der Erfindung, daß anstelle einer Speicherkarte auch eine telemetrische Übertragung beziehungsweise Kommunikation zwischen Defibrillator und Personalcomputer möglich ist oder daß eine bidirektionale Infrarot-Lese/Schreibeinrichtung mit Zwischenspeicher verwendet wird. Des weiteren kann der Datenaustausch zwischen Defibrillator und Personalcomputer induktiv oder mittels Kabel z.B. einem Glasfaserkabel über eine parallele oder serielle Schnittstelle erfolgen.

Der erfindungsgemäße Personalcomputer kann selbstverständlich so ausgebildet sein, daß die beim Einsatz von Defibrillatoren erforderliche Protokollierung und Kommentierung durch einen behandelnden Arzt mittels entsprechender Softwarebausteine erleichtert durchgeführt werden kann.

Mittels der Erfindung gelingt es also, durch konsequente Trennung der Lern- und der Einsatzphase eines neuronalen Netzes die Vorteile desselben zu nutzen, ohne daß die Funktionssicherheit eines Defibrillators hinsichtlich der Auslösung eines Entscheidungskriteriums über die Bereitstellung eines Defibrillatorschocks verschlechtert wird. Die erfindungsgemäße Effizienzprüfung durch ein Defibrillatorsimulationsprogramm innerhalb des Personalcomputers unter Nutzung von Standardreferenzdaten sichert eine außerordentlich schnelle Aussage über die Qualität des vermeintlichen oder tatsächlichen Optimums.

Die Erfindung soll anhand eines Ausführungsbeispiels und mehrerer Figuren näher erläutert werden. Hierbei zeigen:
- Fig. 1: ein Systemblockschaltbild der erfindungsgemäßen Vorrichtung zum effizienten Betreiben eines Defibrillators,
- Fig. 2: ein Blockschaltbild des in der Vorrichtung verwendeten Defibrillators und
- Fig. 3: ein Flußdiagramm zur Erläuterung des prinzipiellen Verfahrensablaufes.

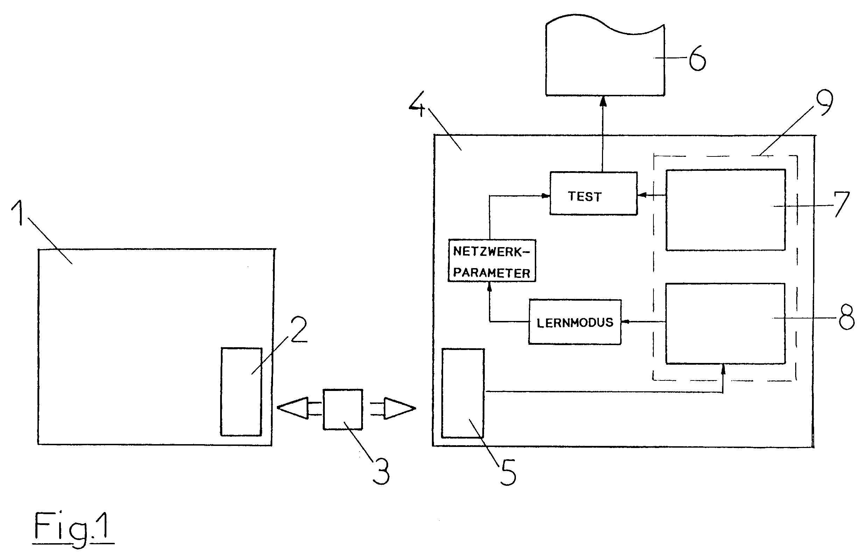

Mit Hilfe der Fig. 1 soll das Zusammenwirken des Defibrillators mit dem Personalcomputer (PC) unter Zuhilfenahme einer auswechselbaren Speicherkarte näher erläutert werden.

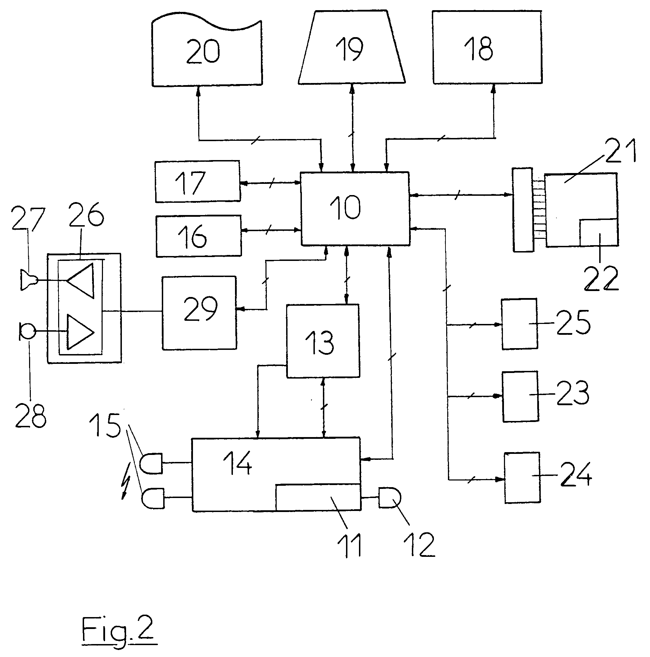

Der Defibrillator 1 verfügt über eine Karten-Lese/Schreibeinrichtung 2, mit deren Hilfe Daten auf eine auswechselbare Speicherkarte 3 geschrieben oder von der Karte 3 gelesen werden können. Bezüglich des Aufbaus des Defibrillators 1 wird auf die nachstehenden Erläuterungen zu Fig. 2 verwiesen.

Der erfindungsgemäße PC 4 verfügt ebenfalls über eine Karten-Lese/Schreibeinrichtung 5 und weist einen Festplattenspeicher 9, einen RAM, eine Eingabeeinrichtung und eine Anzeigeeinheit (nicht gezeigt) auf. Des weiteren steht der PC 4 mit einem Drucker 6 in Verbindung.

Der Personalcomputer 4 verfügt über eine Referenzdatenbank 7 sowie über eine Entwicklungsdatenbank 8, die z.B. auf dem Festplattenspeicher eingerichtet sind.

Mit Hilfe der Kartenlese- und Schreibeinrichtung 2 des Defibrillators 1 werden beim Einsatz desselben erhaltene EKGs aufgenommen und diese zusammen mit den Netzwerkparametern auf die Speicherkarte 3 abgelegt. Die Speicherkarte 3 wird zum Beispiel nach Beendigung des Einsatzes in einem Rettungsfahrzeug der Karten-Lese/Schreibeinrichtung 5 des an zentraler Stelle befindlichen Personalcomputers 4 übergeben. Mit der nicht gezeigten Anzeigeeinheit des Personalcomputers 4 und unter Zuhilfenahme einer Bewertungssoftware kann der Arzt die aufgenommenen EKGs bewerten und entscheiden, welche Daten auf die Entwicklungsdatenbank 8 gelangen.

Diese aktuellen Entwicklungsdaten werden für einen Selbstlernmodus eines neuronalen Netzes im PC 4, welches die Netzwerkparameter des Defibrillators 1 über die Speicherkarte 3 enthält, verwendet. Der externe Selbstlernprozeß selbst ist daher zeitlich unkritisch und kann unter Nutzung bekannter Lernalgorithmen realisiert werden.

Nachdem neue aktualisierte Netzwerkparameter bestimmt wurden, wird mit diesen neuen Netzwerkparametern ein Defibrillatorsimulationsprogramm im Personalcomputer 4 aktiviert, wobei die Leistungsfähigkeit des Netzwerkes mit den aktualisierten Netzwerkparametern anhand von in der Referenzdatenbank 7 gespeicherten Vergleichs- und Referenzdaten überprüft wird.

Im Falle eines positiven Effizienzprüfungsergebnisses, wobei als Kriterium beispielsweise die Zeit bis zur Ableitung einer Ja/Nein-Entscheidung bezüglich des Auslösens eines Defibrillatorschockimpulses herangezogen wird, werden die derart aktualisierten und optimierten Netzwerkparameter über die Karten-Lese/Schreibeinrichtung 5 des Personalcomputers 4 auf die Speicherkarte 3 geschrieben. Die optimierten Netzwerkparameter werden dann mit Rückführung der Speicherkarte 3 zum Defibrillator 1 mit Hilfe der Karten-Lese/Schreibeinrichtung 2 des Defibrillators 1 gelesen und dem dortigen neuronalen Netzwerk zur Verfügung gestellt.

Der interne Aufbau des erfindungsgemäßen Defibrillators soll mit dem Blockschaltbild gemäß Fig. 2 näher erläutert werden.

Der Defibrillator verfügt über einen Mikrocontroller 10 zur Steuerung der Betriebsweise und zur Bewertung einlaufender Elektrokardiogramme. Ein Elektrokardiogrammverstärker 11 steht mit einer Aufnahmeelektrode 12 in Verbindung und kommuniziert über einen Bus mit dem vorerwähnten Mikrocontroller 10.

Über eine spezielle programmierbare Logikeinheit 13 kann der Mikrocontroller 10 einen Defibrillatorpuls auslösen, welcher einen Hochspannungstreiber 14 aktiviert, so daß über Elektroden 15 eine Stimulierung der Herztätigkeit eines Patienten erfolgen kann. Des weiteren steht der Mikrocontroller 10 mit einem Taktgeber 16 und einem Watchdogtimer 17 zur Prozeßkontrolle in Verbindung.

Über eine Bedieneinheit 18 kann eine Funktionssteuerung des Defibrillators vorgenommen oder bestimmte Daten abgefragt und auf einem Display 19 angezeigt werden.

Zur Protokollierung besitzt der Mikrocontroller 10 eine Schnittstelle zum Betreiben eines Druckers 20. Über eine weitere Schnittstelle, z.B. eine standardisierte Schnittstelle PCMCIA, wirkt der Mikrocontroller 10 über eine nicht gezeigte Lese/Schreibeinrichtung mit einer Speicherkarte 21 zusammen. Die Speicherkarte 21 enthält u.a. einen Speicherbereich 22 zur Aufnahme der Netzwerkparameter eines neuronalen Netzes des Defibrillators. Des weiteren steht der Mikrocontroller 10 über einen gemeinsamen Bus mit einem Boot-PROM 23, einem Flash-ROM 24 und einem RAM 25 in Verbindung.

Über einen weiteren Eingang des Mikrocontrollers 10 ist eine Audioaufnahme und Wiedergabe, z.B. zur Protokollierung des Einsatzes des Defibrillators oder zur Abgabe gesprochener Bedienungshinweise, möglich. Hierfür wirkt ein Ein- und Ausgangsverstärker 26 zum Betreiben eines Lautsprechers 27 beziehungsweise des Mikrophons 28 mit einem Datenkompressions- und -dekompressionsschaltkreis 29 zusammen.

Es liegt im Sinne der Erfindung, daß über das Mikrophon 28 eingegebene und mit Hilfe des Kompressionsschaltkreises 29 und dem Mikrocontroller 10 weiterverarbeitete Kommentare oder Hinweise in einem weiteren Speicherbereich der Speicherkarte 21 abgelegt werden können.

Mittels der speziellen programmierbaren Logikeinheit 13 kann über eine Freigabeleitung sichergestellt werden, daß die Betriebssicherheit des Defibrillators unter allen Umständen gewährleistet ist und nicht irrtümlich und aufgrund von Fehlfunktionen ein Elektroschock beziehungsweise eine Ansteuerung der Elektroden 15 ausgelöst wird. Der Mikrocontroller 10 des Defibrillators besitzt des weiteren mehrere Analogdigitalwandler mit einer Auflösung von mindestens 10 bit, welche vorzugsweise auf dem Controllerchip integriert sind.

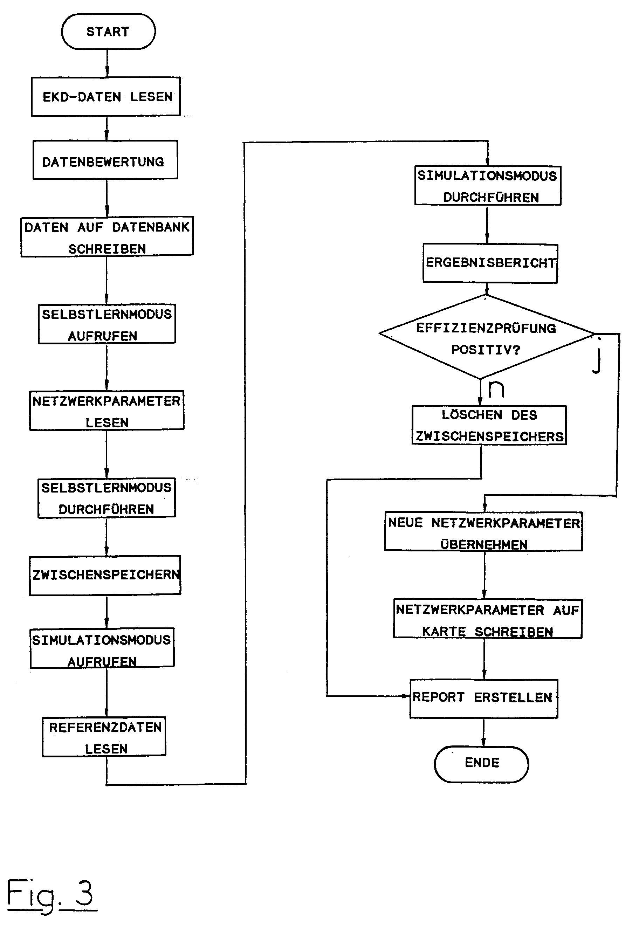

Das erfindungsgemäße Verfahren soll nunmehr unter Verweis auf das Flußdiagramm gemäß Fig. 3 beschrieben werden. Nach dem Start eines im PC 4 (Fig. 1) befindlichen Programms werden die Elektrokardiogramme, welche sich auf der Speicherkarte 3 befinden, ausgelesen und einer Bewertung unterzogen und im Falle einer positiven Bewertung der Entwicklungsdatenbank 8 zugeführt. Nach dem Aufruf eines Selbstlernmodus werden die Defibrillatorparameter, insbesondere die Netzwerkparameter, ebenfalls von der Speicherkarte 3 ausgelesen, und unter Zuhilfenahme dieser Parameter wird eine Selbstlernoperation des im Personalcomputer 4 abgebildeten neuronalen Netzwerkes und eine Zwischenspeicherung der erhaltenen Lernergebnisse durchgeführt.

Das Ziel dieses Lernprozesses ist eine Verbesserung der Netzwerkparamter, um in kürzerer Zeit anhand einlaufender Elektrokardiogramme feststellen zu können, ob eine Herzrhythmusstörung vorliegt und ein Defibrillatorschock ausgelöst werden muß.

In einem nächsten Schritt wird ein Programm zur Simulation der Arbeitsweise eines Defibrillators aufgerufen und dieses Programm mit den neuen, im vorangegangenen Schritt bestimmten Netzwerkparametern abgearbeitet, um feststellen zu können, ob eine Effizienzerhöhung bei der Bewertung einlaufender Elektrokardiogramme möglich ist. Hierfür wird auf Kardiogramme aus der Standardreferenzdatenbank 7 zurückgegriffen.

Nachfolgend wird ein Ergebnisbericht erstellt und gegebenenfalls ausgedruckt, um feststellen zu können, ob die gewünschte Effizienzerhöhung eingetreten ist. Wenn ja, werden die Netzwerkparameter der Speicherkarte 3 überschrieben und ein Revisionsreport erstellt.

Im negativen Fall werden die im Personalcomputer 4 zwischengespeicherten, bei der Simulation verwendeten Daten gelöscht, und es wird ebenfalls ein Report erstellt.

Durch den Ausdruck eines Reportes bei jedem Programmdurchlauf, unabhängig vom erhaltenen Ergebnis, kann eine lückenlose Dokumentation erfolgen, wie dies für medizintechnische Geräte erforderlich ist. Selbstverständlich können die Reporte auch in einem weiteren, internen Speicher des PC abgelegt und zur späteren Auswertung herangezogen werden.

## Patentansprüche

1. Verfahren zum Betreiben eines Defibrillators, wobei der Defibrillator (1) als halbautomatische Einrichtung ausgebildet ist und einen Mikrocontroller (10), fest verdrahtete Speichereinheiten (23, 24, 25), Anzeigeeinrichtungen (19), Mittel zum Aufnehmen eines Elektrokardiogramms (11, 12), eine auswechselbare Speicherkarte (3, 21) zum Abspeichern aufgenommener Elektrokardiogramme, Mittel zur gezielten Erzeugung von Defibrillator-Pulsen (13, 14) sowie Schock-Elektroden (15) aufweist und wobei der Mikrocontroller (10) des Defibrillators (1) zur Analyse des Elektrokardiogramms und zur Freigabe der Defibrillator-Pulse mit einem neuronalen Netzwerk zusammenwirkt,
**dadurch gekennzeichnet,** daß
die Netzwerkparameter des im Vorwärtspfad betriebenen neuronalen Netzwerkes auf der auswechselbaren Speicherkarte (3, 21, 22) abgelegt sind, wobei über die Speicherkarte (3, 21) eine temporäre Kommunikation des Defibrillators (1) mit einem Personalcomputer (4) erfolgt,
- der Personalcomputer (4) die auf der Speicherkarte (3, 21) befindlichen Elektrokardiogramme dann in eine interne Entwicklungs-Datenbank (8) übernimmt, wobei die Entwicklungsdatenbank (8) mit den übernommenen Daten aktualisiert wird, wenn festgestellt wird, daß aus dem Verlauf des jeweiligen Elektrokardiogramms die Erzeugung von Defibrillator-Pulsen erforderlich ist,
- mit den aktualisierten Daten der Entwicklungs-Datenbank (8) die Netzwerkparameter des neuronalen Netzes durch einen Lernprozeß des im Rückwärtspfad betriebenen Netzes im Personalcomputer (4) optimiert werden,
- und mit den neuen, optimierten Netzwerkparametern mittels eines Defibrillator-Simulationsprogramms und unter Nutzung von auf einer internen Referenz- beziehungsweise Standard-Datenbank (7) des Personalcomputers (4) gespeicherten Vergleichsdaten eine Effizienzprüfung der optimierten Netzwerkparameter durchgeführt wird, wobei im Ergebnis einer positiven Effizienzprüfung mit den optimierten Netzwerkparametern ein Überschreiben und Aktualisieren der Netzwerkparameter auf der auswechselbaren Speicherkarte (3, 21) erfolgt, und daß die auf der Speicherkarte (3, 21) befindlichen Daten vom Defibrillator (1) übernommen werden.

2. Verfahren nach Anspruch 1,
**dadurch gekennzeichnet,** daß
der Zyklus aus Übergabe/Übernahme von Elektrokardiogrammen und Netzwerkparametern, externem Optimieren der Netzwerkparameter und Rückübertragung optimierter Netzwerkparameter nach jedem Einsatz des Defibrillators wiederholt wird.

3. Verfahren nach Anspruch 1,
**dadurch gekennzeichnet,** daß
mehrere Defibrillatoren über auswechselbare Speicherkarten mit einem einzigen Personalcomputer kommunizieren.

4. Verfahren nach einem der vorangegangenen Ansprüche,
**dadurch gekennzeichnet,** daß
die gesamte Betriebssoftware des bzw. der Defibrillatoren oder Teile davon über die auswechselbare Speicherkarte änderbar ist.

5. Vorrichtung zum Betreiben eines Defibrillators, umfassend
einen halbautomatischen Defibrillator mit einem Mikrocontroller (10), fest verdrahteten Speichereinheiten (23, 24, 25), Anzeigeeinrichtungen (19), einer Bedieneinheit (18), Mittel zum Aufnehmen eines Elektrokardiogramms (11), einer auswechselbaren Speicherkarte (3, 21) zur Aufnahme von Elektrokardiogrammen sowie Mittel zur Erzeugung von Defibrillator-Pulsen (13, 14), wobei der Mikrocontroller (10) zur gezielten Erzeugung von Defibrillator-Pulsen unter Nutzung eines neuronalen Netzwerkes die Elektrokardiogramme bewertet und Schockelektroden (15) ansteuert,
**dadurch gekennzeichnet,** daß
über die auswechselbare Speicherkarte (3, 21), die zusätzlich die Netzwerkparameter des neuronalen Netzes enthält, eine temporäre Verbindung zu einem Personalcomputer (4) besteht, wobei der Personalcomputer (4) eine Karten-Lese/Schreibeinrichtung (5) und interne Datenbanken (7, 8), Mittel zur Simulation der Betriebsweise des Defibrillators (1) und eine Anzeigeeinheit zum Darstellen und Bestätigen der Übernahme von auf der Speicherkarte (3, 21) befindlichen Elektrokardiogrammen in eine der internen Datenbanken (8) aufweist.

6. Vorrichtung nach Anspruch 5,
**dadurch gekennzeichnet,** daß
der Personalcomputer (4) eine Entwicklungsdatenbank (8) zur Übernahme bestätigter Elektrokardiogramme von der Speicherkarte (21) und eine Referenz- oder Vergleichsdatenbank (7) mit bekannten Standarddaten enthält, wobei eine Optimierung der Netzwerkparameter durch einen Selbstlernprozeß unter Nutzung der übernommenen Elektrokardiogramme durchgeführt und die aktualisierten Netzwerkparameter mit Hilfe der Mittel zur Simulation der Betriebsweise des Defibrillators (1) auf Effizienz geprüft und im positiven Fall mittels der Karten-Lese/Schreibeinrichtung (5) die aktualisierten Netzwerkparameter auf die Speicherkarte (3) geschrieben und über eine Standardschnittstelle beziehungsweise eine weitere Karten-Lese/Schreibeinrichtung (2) in den Defibrillator (1) übernommen werden.

7. Vorrichtung nach Anspruch 5,
**dadurch gekennzeichnet,** daß
der Mikrocontroller (10) über eine Datenkompressions-Dekompressionseinheit (29) mit einer Audio-Aufnahme und -wiedergabeeinrichtung (26, 27, 28) in Verbindung steht, wobei die komprimierten Audioinformationen zusätzlich auf der auswechselbaren Speicherkarte (21) abgelegt und zur Unterstützung der Auswertung gespeicherter Elektrokardiogramme ebenfalls zum Personalcomputer (4) übertragen werden.

8. Vorrichtung nach Anspruch 5,
**dadurch gekennzeichnet,**
daß zusätzlich oder anstelle der auswechselbaren Speicherkarte (3, 21) eine weitere, mindestens temporäre Verbindung zum Datenaustausch zwischen Defibrillator (1) und Personalcomputer (4) vorgesehen ist.

9. Vorrichtung nach Anspruch 8,
**dadurch gekennzeichnet,**
daß die weitere, mindestens temporäre Verbindung eine telemetrische Datenübertragung ist.

10. Vorrichtung nach Anspruch 8,
**dadurch gekennzeichnet,**
daß die weitere, mindestens temporäre Verbindung aus einer bidirektionalen Infrarot-Lese/Schreibeinrichtung mit Zwischenspeicher besteht.

11. Vorrichtung nach Anspruch 8,
**dadurch gekennzeichnet,**
daß die weitere, mindestens temporäre Verbindung aus einem Induktionssender/Empfänger mit Zwischenspeicher besteht.

12. Vorrichtung nach Anspruch 8,
**dadurch gekennzeichnet,**
daß die weitere, mindestens temporäre Verbindung mittels paralleler oder serieller Schnittstelle über Kabel hergestellt ist.
